# EUROPEAN PATENT APPLICATION

(11) **EP 2 476 665 A1**
(43) Date of publication of application: **18.07.2012**
(21) Application number: 11151066.5
(22) Date of filing: 17.01.2011
(51) Int. Cl.: C07C 213/02, C07C 231/12, C07C 235/34, C07C 215/54

(54) **Process for the preparation of N,N-diisopropyl-3-(2-hydroxy-5-methylphenyl)- 3-phenyl propylamine and its salts starting from a novel intermediate**

(71) Applicant: Cambrex Profarmaco Milano S.r.l., 20155 Milano (IT)
(72) Inventor: Gianolli, Edoardo, 20067 Paullo (MI) (IT); Giannini, Elios, 20155 Milano (IT); Bigini, Laura, 20047 Brugherio (MI) (IT); Piccolo, Oreste, 23896 Sirtori (LC) (IT); Holmberg, Pär, 20155 Milano (IT); Lundholm, Tommy, 20155 Milano (IT)
(74) Representative: Minoja, Fabrizio

(57) **Abstract**

The invention concerns an improved process for the preparation of tolterodine (N,N-diisopropyl-3-(2-hydroxy-5-methylphenyl)-3-phenyl propylamine) and its salts, in particular for the preparation of the tartrate salt, and more in particular for the (+)-(R) enantiomer of tolterodine L-tartrate, starting from a novel intermediate, N,N-diisopropyl-3-(2-hydroxy-5-methylphenyl)-3-phenyl-2-propenamide which can be used as pure Z or E isomer or as a mixture of Z and E isomers.

## Description

The invention concerns an improved process for the preparation of tolterodine (N,N-diisopropyl-3-(2-hydroxy-5-methylphenyl)-3-phenyl propylamine) and its salts, in particular for the preparation of the tartrate salt, and more particularly for the (+)-(R) enantiomer of tolterodine L-tartrate, starting from a novel intermediate, N,N-diisopropyl-3-(2-hydroxy-5-methylphenyl)-3-phenyl-2-propenamide, which can be used as pure Z or E isomer or as a mixture of Z and E isomers.

### Background of the invention

(R)-N,N-Diisopropyl-3-(2-hydroxy-5-methylphenyl)-3-phenyl propylamine, known as (R)- Tolterodine, is an anti-cholinergic agent, useful in the treatment of urinary incontinence presently marketed in form of its salt with L-tartaric acid.

US 6,310,103 discloses the corresponding enantiomer (S)-N,N-diisopropyl- 3-(2-hydroxy-5-methylphenyl)-3-phenyl propylamine ant its salts as drugs for use in the treatment of disorders of the urinary and gastrointestinal tracts.

A large number of methods for producing (N,N-diisopropyl-3-(2-hydroxy-5-methylphenyl)-3-phenyl propylamine) (I), its enantiomers and the corresponding salts have already been described. Many of them make use of starting products hardly available or dangerous reagents or the synthesis requires a high number of steps, so that the preparation is troublesome or may be not suitable on larger scale.

The preparation of (R,S)-(I) is described in US 5,382,600 where the product was recovered in numerous steps, long reaction time, and low overall yields by employing the intermediate 3,4-dihydro-6-methyl-4-phenyl-2H-benzopyran-2-one (VI). Furthermore, the use of expensive and hazardous reagents and solvents like methyl iodide, lithium aluminium hydride, and boron tribromide also renders this process unsuitable and hazardous on a commercial scale. The corresponding (R)-enantiomer, (R)-(I) was obtained by selective diastereomeric crystallization using L-(+)-tartaric acid as resolving agent in 95% ethanol and following recovering from the salt, if required. As a matter of fact, the L-tartrate salt of (R)-(I) has been directly marketed as suitable pharmaceutical form.

In the same patent, a second process is described, starting from (VI), that is transformed in a few steps into 3-[(2-methoxy-5-methylphenyl)-3-phenyl-propionic acid. The following chlorination of the acidic intermediate with SOCl₂ and the reaction with diisopropylamine permits to obtain the N,N-diisopropyl-3-(2-methoxy-5-methylphenyl)-3-phenyl-propamide which is reduced by means of reductive agents such as LiAlH₄ or sodium dihydro-bis-methoxyethoxy aluminate (Vitrid^{™}). The product (I) is finally obtained by deprotecting the methyl ether group with boron tribromide. It is clear that also this method is not suitable for application on industrial scale.

US 6,822,119, US 7,355,077 and WO 2008020332 disclose different methods for the preparation of (I). All of them comprise the conversion of compound (VI) into the corresponding 3-(2-(OR)-5-methylphenyl)-3-phenyl-propan-1-ol intermediate, where OR is hydroxy, methoxy or benzyloxy group, the subsequent transformation of primary alcohol function into a suitable sulphonate able to react with diisopropylamine. Finally, the compound (I) is obtained by removing the protection of phenolic OH group, isolated in the form of the corresponding hydrobromide or hydrochloride salt and purified by crystallization. Where described, the resolution is carried out by using L-tartaric acid in ethanol or in a mixture of methanol/acetonitrile.

Also these processes involve a large number of steps, giving poor yields.

A further problem associated with these procedures is that dimeric impurities are generally associated with compound (I) and are difficult to remove as reported in US 7,538,249. These impurities are formed during the reaction of the sulphonate intermediates with diisopropylamine. In order to obtain a product (I) suitable for the resolution step it is necessary to carry out an efficient purification of crude (I) which determines a decrease in the final yield.

US 5,922,914 discloses a novel intermediate, 3,4-dihydro-6-methyl-4-phenyl-2H-benzopyran-2-ol (IX), and an improved process for the preparation of (I) which involves the following steps: a) the reaction of trans-cinnamic acid with 4-methyl-phenol in acidic medium to give the above mentioned lactone (VI); b) the reduction of (VI) with diisobutyl aluminium hydride (DIBAL) to yield the corresponding benzopyran-2-ol; c) the reductive amination of the latter compound with diisopropylamine, in the presence of Pd/C catalyst in methanol. The resolution is finally carried out using ethanol or a mixture methanol/acetone with L-(+)-tartaric acid.

This method of synthesis reduces the number of steps but it seems not to be commercially feasible since it involves the use of DIBAL as reducing agent which is an expensive and hazardous reagent.

The derivative intermediate compound N,N-diisopropyl-3-(2-Hydroxy-5-methylphenyl)-3-phenyl propionamide (V) as racemate was already described in the literature as, for example, in US 6,310,248, US 7,119,212, US 7,335,793, IN 2003CH01028, CN 101445462, but has been obtained by means of processes different from the one object of the present invention. The examples, reported in the literature to obtain such intermediate, give low yields or need processes with particular protections or multi step processes so that it was not previously considered useful intermediate to synthesize (I). In some of these patents, enantioenriched (V) was quoted as possible intermediate but no detailed description of this compound was previously found.

On the basis of the reported state of the art, there is still a need for finding improved processes for the preparation of (I) whereby the above problems can be alleviated and a product with improved purity and economies in the process can be obtained.

### Description of the Invention

It has now surprisingly been found that a compound having formula (II) 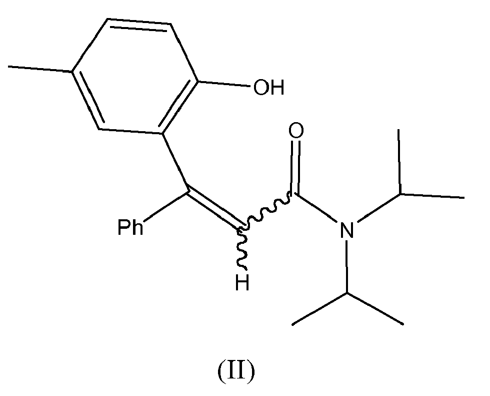 can be prepared in high yield from easily available products, by means of a simple one-step process which allows to avoid the use of hardly available starting products, reactants and reaction conditions troublesome for industrial production. Compound II can be transformed into the corresponding compound (V) as racemate or as enantioenriched compound in a nearly quantitative yield.

Compound (II), as pure Z or E isomer or as isomeric mixture of Z and E isomers, may be advantageously obtained by the Mizoroki-Heck reaction, contrary to what could be expected from the prior art, as better described later. The Z or E form, or mixtures thereof, can be obtained by modulating appropriately the reaction conditions and the related work-up because the two isomers have different solubility characteristics and a different stability with the temperature and the use of organic solvents.

Accordingly, in addition to the compound II, the invention refers also to a process for the preparation of N,N-diisopropyl-3-(2-hydroxy-5-methylphenyl)-3-phenyl-propylamine (I) starting from said compound (II) either as pure Z or E isomer or as a mixture of Z and E isomers.

The invention also concerns a process for the preparation of the intermediate (II) by means of the Mizoroki-Heck reaction, (a detailed overview of this reaction is reported in "The Mizoroki-Heck Reaction", ed. M.Oestreich, Wiley 2009)
which comprises:
1) reacting a 2-halo-4-methyl-phenol of formula (III) 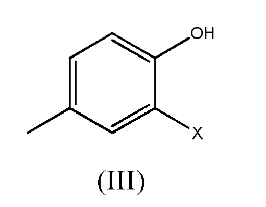 wherein X is Cl, Br or I
   with N,N-diisopropyl-3-phenyl-2-propenamide (IV) 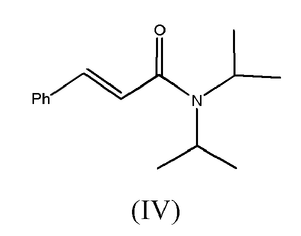 in the presence of a base and of a suitable catalyst, preferably a palladium catalyst, to obtain the compound of formula (II) as a pure Z or E isomer or as a mixture of Z and E isomers, depending on the reaction and work-up conditions, the formation and/or the interconversion of both isomers being affected by the experimental conditions;
2) optional additional purification of crude compound (II), if necessary, for instance by selective crystallization, if a pure isomer is required.

As to step 1), it should be pointed out that the Mizoroki-Heck reaction, previously described on unsaturated compounds, such as unsaturated cinnamide esters (J. Org. Chem. 2007, 72, 6056-86059), and on 3-arylacrylamides (Bull. Korean Chem. Soc. 1999), has never been used on a N,N-dialkyl substituted amide. Furthermore, the excellent result observed in the present invention was unexpected because it has been reported that this type of reaction is not easily applicable on an unsaturated amide.

Starting from this novel, purified or crude isolated intermediate or from the same compound without isolating it from the reaction mixture, it is possible to carry out the synthesis of (I), by a process comprising:
i) reducing the intermediate (II) with a suitable reducing agent to obtain compound (V) as racemic mixture or enantiomerically enriched in (R) or (S) enantiomer; 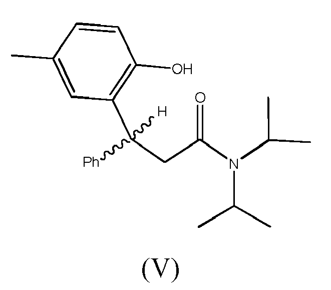
ii) reducing the amide (V) to the corresponding amine (I) as racemic mixture or enantiomerically enriched in (R) or (S) enantiomer; 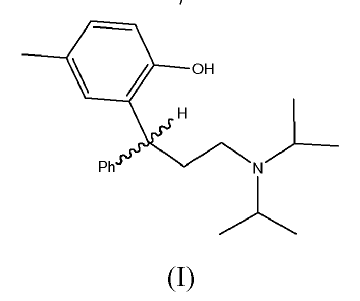
iii) isolating the compound (I) as a salt by adding a suitable inorganic or organic acid, preferentially tartaric acid and more preferentially enantiomerically pure L- or D- tartaric acid, using the L-enantiomer to obtain the (R)-(I) tartrate salt and the D-enantiomer to obtain the (S)-(I) tartrate salt;
iv) optionally, converting the racemic or nearly racemic (R,S)-(I) L-tartrate or enantiomeric enriched (R)-(I) L-tartrate into the enantiopure (R) enantiomer as L-tartrate salt by selective crystallization.

Alternatively, step iv) is carried out on the D-tartrate salt if the enantiopure (S)-(I) enantiomer is desired.

The intermediate (II) may be reduced with an almost quantitative yield in mild conditions to (R,S)-(V) using H₂ or an hydrogen donor such as sodium hypophosphite in the presence of Pd/C as catalyst. Then, (R,S)-(V) can be converted into (I) by reduction with Vitride: the reaction product may be isolated as a salt by adding an inorganic or organic acid. The compound (I) is preferably isolated directly as racemic L-tartrate or enantiomerically enriched L-tartrate salt so avoiding, if the preparation of (R)-(I)- L-tartrate is desired, the intermediate formation of a different salt, with a clear advantage over the prior art. In fact, the quality of the isolated product (I) as tartrate is particularly suitable for the subsequent crystallization to obtain (R)-(I) in enantiomerically pure form by conventional crystallization methods.

Alternatively, the intermediate (II) can be reduced in two steps to the enantiomerically enriched form of (I) having enantiomeric excess up to 91%, if the C=C reduction occurs in the presence of a chiral catalyst; enantiomeric excess and absolute configuration depending not only on the used chiral catalyst but also on the E or Z isomer used as starting material.

Thanks to this invention that is characterized by the formation of novel (II) intermediate, the process to provide (I) as base and as salts, preferentially as the tartrate salt, appears simple, efficient and flexible to the requirements relating to product configuration.

The overall process for the preparation of (I) is easy to scale industrially and gives the desired product in good yield and quality.

### Detailed description of the invention

The Mizoroki-Heck reaction used for the preparation of the intermediate (II) can be carried out using commercially available starting materials (III) and (IV), using a ratio (IV)/(III) which is in the range 2.5/1 to 0.9/1, preferably 1.5/1 - 1/1. It is performed in the presence of a solvent, preferentially an organic solvent with medium or low polarity such as, for example, 2-methyl-THF, dioxane or toluene. Typically, the ratio between amount of solvent and (III) is 2-10/1 w/w. Furthermore, the reaction is performed in the presence of an organic or inorganic base and, preferentially, in the presence of sterically hindered organic bases such as N,N-dicyclohexylmethylamine. The stoichiometric ratio between the base and (III) is typically in the range from about 1/1 to 1.5/1, preferentially from 1.3/1 to 1/1. The Mizoroki-Heck reaction requires the presence of a catalyst, preferentially a homogeneous Pd(0) catalyst, prepared in situ or preformed in a separate vessel and added to the reaction mixture, such as for example Pd(0)L₂ + R₃P or Pd(0)(PR₃)₂. A suitable Pd(0)L₂ is for example Pd₂(dba)3 where dba is dibenzylideneacetone. When Pd in a different oxidation state is added to the reaction mixture, such as for example Pd(OAc), it is necessary that it is reduced *in situ* to obtain the active Pd(0) catalyst. The most suitable phosphines for this reaction should be the hindered electron rich phosphines such as, for example, t-butylphosphine, di-t-butyl(2,2-diphenyl-1-methyl-1-cyclopropyl)phosphine (c-BRIDP), di(1-adamantyl)-*n*-butylphosphine. The stoichiometric ratio between (III) and the catalyst should be in the range from about 100:1 to 10000:1, preferably from 500:1 to 5000:1 and more preferably from 1000:1 to 3000:1.

The reaction temperature can affect the isomeric ratio between the E and Z isomer of (II) and can be modulated to obtain one of the two isomers in pure form or in larger excess. Temperatures lower than 100°C usually afford higher contents of E isomer, temperatures higher than 100°C afford higher amounts of Z isomer.

The purity of the E or Z isomer may be increased by simple crystallization in a suitable solvent. For example, the highly pure E isomer may be obtained by crystallization in toluene where the Z isomer is more soluble, while the highly pure Z isomer may be obtained directly from the synthesis, by fine tuning the experimental conditions.

The E or Z isomers of novel product (II) have been characterized by HPLC [Column Agilent Zorbax RX8 250x4.6 mm; Mobile phase = 1.54 g CH₃COONH₄ + 500 ml water + 1,0 ml CH3COOH+ 500 ml CH₃CN; Flow= 1ml/min. wave length: 220 nm. Loop volume 10 µL; RT 18.2 (E isomer),18.9 (Z isomer)], melting point (DSC): 161-164°C ((E)-isomer), 145-151°C ((Z)-isomer), ¹H NMR (300 MHz, CDCl_{3,} δ in ppm) (E isomer): 0.93 (d, 6H), 1.39 (d, 6H), 2.23 (s, 3H), 2.72 (br s, 1H), 3.35 (m, 1H), 4.27 (m, 1H), 6.25 (s, 1H), 6.78 (d, 1H), 6.86 (s, 1H), 7.02 (d, 1H), 7.30 (m, 5H); ¹H NMR (300 MHz, deuterated DMSO, δ in ppm) (Z isomer): 0.89 (d, 6H), 1.18 (d, 6H), 2.16 (s, 3H), 2.72 (br s, 1H), 3.25 (m, 1H), 4.12 (m, 1H), 6.48 (s, 1H), 6.69 (d, 1H), 6.89 (s, 1H), 6.96 (d, 1H), 7.15 (m, 1H),7.25 (m, 5H), 8.95 (s, 1H).

The reduction of product (II) to (R,S)-(V) can be performed by means of H₂ or with hydrogen donors, such as sodium hypophosphite, in the presence of wet 3%, 5% or 10% Pd/C as catalyst, using a solvent selected from the group consisting of THF, methyl-THF or alcohols such as methanol and ethanol, optionally adding 0.5-10% of water, and using a pressure which ranges from atmospheric pressure to 10 bar, the ratio between amount of solvent and (II) being in the range 5/1-20/1 w/w.

The ratio between the catalyst and (II) is in the range of 1/300-1/5, preferably 1/200-1/10 w/w.

The temperature of the hydrogenation is in the range of 30-100°C and preferentially 40-80°C. The reaction time is in the range of 0.1-5 h, typically 0.5-2h. The yield of isolated (R,S)-(V) is > 90%, and usually >95%.

It is also possible to reduce directly the Mizorobu-Heck reaction mixture containing the crude, not isolated, compound (II) in order to obtain in one-pot the saturated amide (V) that may be purified, if necessary, by crystallization.

When an asymmetric reduction is performed, the enantiomeric excess of (V) is also a function of the isomeric purity of the unsaturated product (II), so that it will be proper to proceed through the isolation of the latter compound. To carry out such type of reaction, a few known catalysts suitable for asymmetric reduction of unsaturated olefins, e.g. homogeneous chiral catalyst based on Ruthenium, Rhodium, Iridium, Cobalt, Copper, Zinc with suitable chiral ligands, can be used. The outcome of this reaction is affected either by the used catalyst or by the isomeric purity and type of compound (II).

For example, using a Ru catalyst with a chiral phosphine such as SegphosT^{™} (Takasago) and working on pure Z or E isomer of (II), enantioenriched (V) is obtained with an e.e. up to 91.6% with a nearly quantitative conversion. It was observed that the absolute configuration and the e.e. of (V) may be affected by the type of isomer if the same chiral catalyst is used.

The reduction of the saturated amide (V) to (I) is carried out, as described in the literature, using VITRIDE^{™}; according to a preferred procedure, the compound (I) may be isolated directly as (L) tartrate salt. This product has a ratio between (R)-(I) and (S)-(I) in the range of 50/50 or 60/40 and high chemical purity, greater than 97%, and for this reason it is suitable for further enrichments by crystallization, performed in alcoholic solvents or in mixture containing alcohols, following known procedures.

The invention will be further illustrated by the following examples.

### Example 1

### Preparation of N,N-diisopropyl-3-(2-hydroxy-5-methylphenyl)-3-phenyl-2-propenamide (II)

In a vessel, under nitrogen atmosphere, *N*,*N-*diisopropylcinnamamide (278 g, 1.2 mol), 2-bromo-4-methylphenol (150 g, 0.8 mol), N,N dicyclohexylmethylamine (204 g, 1.0 mol) and 2-methyltetrahydrofuran (1.5 L) are loaded and the temperature is adjusted to 80°C. The catalyst, bis(tri-tert-butylphosphine)Pd, (0.82 g, 2mmol) is added to the vessel and the reaction mixture is stirred until complete conversion is reached (time about 4 h). An aqueous solution of hydrochloric acid (2 w/w-%) is added until pH =1 and the mixture is cooled at 55°C. The phases separate, the water phase is discarded and the organic phase is washed again with water. The solution is then concentrated under vacuum at ≤ 50°C to remove water by azeotropic distillation of water and 2-methyltetrahydrofuran mixture and finally treated with celite and charcoal at 50°C under stirring for 30' and filtered. The filter cake is washed with 2-methyltetrahydrofuran. The solvent is then removed under vacuum at ≤ 50°C and the residue is charged with toluene (0.75 L) and temperature adjusted to about 80°C to obtain a homogeneous solution. Finally the mixture is slowly cooled to 0 - 5°C and the product (II) isolated by filtration and dried under vacuum (231 g, 85%, E/Z isomeric ratio 75/25, purity >98%).

### Examples 2 and 3

### Preparation of N,N-diisopropyl-3-(2-hydroxy-5-methylphenyl)-3-phenyl-2-propenamide (II)

Following the procedure of example 1 but heating the toluene mixture at 105°C for 2 or 4.5h, the product (II) isolated by filtration and dried under vacuum is recovered with c.y. 85% (E/Z isomeric ratio 75/25) or with c.y. 68% (E/Z isomeric ratio 49/51).

### Example 4

### Preparation of N,N-diisopropyl-3-(2-hydroxy-5-methylphenyl)-3-phenyl-2-propenamide (II)

Following the procedure of example 1 but isolating the product (II) directly by concentration of 2-methyl-tetrahydrofuran mixture at ≤ 50°C, is recovered with c.y. 74% (E/Z isomeric ratio 99/1).

### Example 5

### Preparation of N,N-diisopropyl-3-(2-hydroxy-5-methylphenyl)-3-phenyl-2-propenamide (II)

A mixture of 2-bromo-4-methylphenol (375 g, 2.0 mol), N,N-diisopropylcinnamide (695g, 3.0 mol) and N,N-dicyclohexylmethylamine (612 g, 3.0 mol) is added to 5 litre feed vessel and mixed together under stirring. To the mixture 1 L of degassed 2-methyltetrahydrofuran (2-MeTHF) is then added. To a second feed vessel 2.5 L of degassed 2-MeTHF are charged, followed by tri-tertbutylphosphine (8.1 g, 40mmol) and Pd₂(dba)₃ (9.2 g, 10 mmol) dissolved in a small amount of degassed 2-MeTHF. After priming the continuous flow reactor system with 2-MeTHF, setting the pressure to 7 bars, the plate reactor is heated conventionally with heated oil to reaction temperature, 150-155°C. When the reaction temperature has been reached, the bottom valves of both the feed vessels are opened and the combined flow is directed to the plate reactor. The flow rate is adjusted to correspond to a residence time in the plate reactor of 3 minutes; i.e. flow rate is set to 24 ml/min and the residence volume in the reactor is 73 ml. The temperature of the reaction mixture inside the plate reactor is measured with 3 independent thermo couples and displayed on computer screen all through the experiment. The outlet is continuously collected in two separate receiver vessels and they are discharged after completed experiment. The first vessel is in duty up the point of acceptable level of conversion and the second to collect the batch thereafter. After the usual work-up the product (II) is recovered as nearly pure Z isomer with c.y. 60%

### Examples 6-20

### Preparation of N,N-diisopropyl-3-(2-hydroxy-5-methylphenyl)-3-phenyl-2-propenamide (II)

The preparation of compound (II) is performed working at different reaction conditions and with different catalysts. All experiments are run with 1.5 equivalents of 2-bromo-cresol and 1.5 equivalents of base unless otherwise stated. Conversion and reaction profile is based on HPLC peak area %. (Tables 1 and 2)

**Table 1**

| Example | Pd Source | Additives Solvent | Time (h) | Temp (°C) | Conv. (%) | 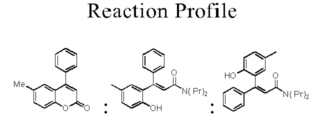 | | |
|---|---|---|---|---|---|---|---|---|
| 6^{a} | Pd₂(dba)₃ 0.02 eq | [(CH₃)₃C]P Me(Cy)₂N Dioxane | 18 | 80 | 82 | 11 : | 71 : | 17 |
| 7 | Pd(OAc)₂ 0.02 eq | cBRIDP Me(Cy)₂N Toluene | 24 | 80 | 100 | 35 : | 59 : | 6 |
| 8 | Pd(OAc)₂ 0.02 eq | cBRIDP Me(Cy)₂N Toluene | 18 | 70 | 95 | 23 : | 71 : | 6 |
| 9 | Pd₂(dba)₃ 0.02 eq | cBRIDP Me(Cy)₂N Dioxane | 20 | 75 | 91 | 1 : | 79 : | 11 |
| 10 | Pd₂(dba)₃ 0.02 eq | cBRIDP Me(Cy)₂N 2-Me-THF | 6 | 80 | 70 | 0 : | 73 | : 4 |
| 11^{a} | Pd₂(dba)₃ 0.02 eq | cBRIDP Me(Cy)₂N Dioxane | 5 | 80 | 100 | 1 : | 95 | : 4 |
| 12^{a} | Pd(OAc)₂ 0.01 eq | cBRIDP Me(Cy)₂N Toluene | 2 | 80 | 77 | 3 : | 94 : | 3 |

| | | | | | | | | |
|---|---|---|---|---|---|---|---|---|
| ^{a} 1.5 equivalents of *N*,*N* diisopropylcinnamide and 1.3 equivalents of base. | | | | | | | | |

**Table 2**

| Entry | Pd Source | Additives Solvent | Time (h) | Temp (°C) | Conv. (%) | 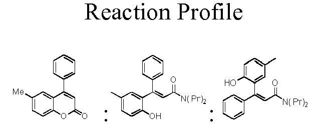 | | |
|---|---|---|---|---|---|---|---|---|
| 13 | Pd(OAc)₂ 0.02 eq | cBRIDP Me(Cy)₂N DMA | 18 | 100 | 100 | 46 | : 42 : | 12 |
| 14 | Pd₂(dba)₃ 0.02 eq | cBRIDP Me(Cy)₂N^{a} Dioxane | 18 | 80 | 89 | 2 | : 87 : | 11 |
| 15 | Pd₂(dba)₃ 0.02 eq | cBRIDP Mₑ(Cy)₂N^{b} Dioxane | 18 | 80 | 89 | 2 | : 87 : | 11 |
| 16^{c} | Pd₂(dba)₃ 0.02 eq | cBRIDP Me(Cy)₂N^{d} Dioxane | 18 | 80 | 23 | 6 | : 84 : | 10 |
| 17^{e} | Pd₂(dba)₃ 0.01 eq | cBRIDP Me(Cy)₂N Dioxane | 18 | 80 | 62 | 8 | : 72 : | 20 |
| 18^{f} | Pd₂(dba)₃ 0.02 eq | cBRIDP Me(Cy)₂N Dioxane | 2 min | 150 (MW) g | 87 | 3 | : 66 : | 31 |
| 19^{f} | Pd₂(dba)₃ 0.02 eq | [(CH₃)₃C]P^{g} Me(Cy)₂N Dioxane | 2 | 80 | 100 | 0 | : 95 | : 5 |
| 20 | Pd₂(dba)₃ 0.02 eq | Me(Cy)₂N Dioxane | 2 min | 150 (MW) g | 17 | 26 : | 23 | : 51 |

| | | | | | | | | |
|---|---|---|---|---|---|---|---|---|
| ^{a}2 equivalents. ^{b}1.1 equivalents. ^{c}2 equivalents 2-bromo-cresol. ^{d}2 equivalents. ^{e}1.1 equivalents of *N*,*N*-diisopropylcinnamide. ^{f} 1.5 equivalents of *N*,*N-*diisopropylcinnamide. ^{g} heating by using microwaves (MW) | | | | | | | | |

### Examples 21-28

### Preparation of N,N-diisopropyl-3-(2-hydroxy-5-methylphenyl)-3-phenyl-2-propenamide (II)

The preparation of compound (II) is performed working at different reaction conditions and with the same Pd catalyst precursor at different concentration. All experiments are run with 1.3 equivalents of base.

Conversion and reaction profile is based on HPLC peak area % (Table3).

**Table 3**

| Example | Pd Source | Additives Solvent | Time (h) | Temp (°C) | Conv. (%) | 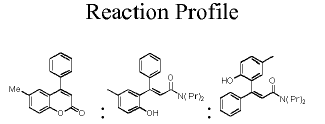 | | |
|---|---|---|---|---|---|---|---|---|
| 21^{a} | Pd₂(dba)₃ 0.02 eq | [(CH₃)₃C]P^{b} Me(Cy)₂N Dioxane | 2 min | 150 (MW) | 100 | 3 | : 51 | : 46 |
| 22^{c} | Pd₂(dba)₃ 0.01 eq | [(CH₃)₃C]P^{d} Me(Cy)₂N Dioxane | 2 | 80 | 100 | 14 | : 78 | : 8 |
| 23^{a} | Pd₂(dba)₃ 0.02 eq | [(CH₃)₃C]P^{b} Me(Cy)₂N 2-Me-THF | 2½ | 80 | 100 | 0 | : 93 : | 7 |
| 24^{a} | Pd₂(dba)₃ 0.02 eq | [(CH₃)₃C]P^{b} Me(Cy)₂N Dioxane | 4 min | 130 (MW) | 100 | 2 | : 66 | : 32 |
| 25^{a} | Pd₂(dba)₃ 0.01 eq | [(CH₃)₃C]P^{d} Me(Cy)₂N Dioxane | 2 min | 150 (MW) | 100 | 3 | : 50 | : 47 |
| 26^{a} | Pd₂(dba)₃ 0.01 eq | [(CH₃)₃C]P^{d} Me(Cy)₂N 2-Me-THF | 2 min | 150 (MW) | 100 | 4 | : 45 | : 51 |
| 27^{a,e} | Pd₂(dba)₃ 0.02 eq | [(CH₃)₃C]P^{b} Me(Cy)₂N Dioxane | 2 h | 80 | 100 | 0 | : 88 | : 51 |
| 28^{a} | Pd₂(dba)₃ 0.005 eq | [(CH₃)₃C]P^{f} Me(Cy)₂N 2-Me-THF | 2 min | 150 (MW) | 100 | 3 | : 51 | : 46 |

| | | | | | | | | |
|---|---|---|---|---|---|---|---|---|
| ^{a}1.5 equivalents of *N*,*N*-diisopropylcinnamide. ^{b}0.04 equivalents. ^{c}1.3 equivalents of *N*,*N* diisopropylcinnamide. ^{d}0.02 equivalents. ^{e} 20 g scale with respect to 2-bromo-cresol. ^{f}0.01 equivalents. | | | | | | | | |

### Example 29

### Preparation of (±)N,N-diisopropyl-3-(2-hydroxy-5-methylphenyl)-3-phenyl-2-propanamide (R,S)-(V)

Compound (II) (168 g, 0.499 mol), 2-Me-THF (2.0 L) and water (40 mL) are loaded into a 3 L round bottomed flask, fitted with a mechanical stirrer. The flask is washed three times with nitrogen, 5% Pd/C (18g, 50% wet) is added and the reaction mixture is stirred under hydrogen atmosphere (1 bar) at 55°C. After 2.5 hrs the HPLC showed complete conversion. The reaction is filtered, at 55°C, trough a pad of celite and the filter is washed with wet, warm 2-MeTHF (180 mL). The filtrate is concentrated to a volume of about 350 mL under reduced pressure, toluene (720 mL) is added and 350 mL of solvent is distilled off, under reduced pressure. The mixture is cooled to 0-5°C and stirred for 1hr. Then the solid is filtered, washed with cold toluene (2 x 60 mL) and dried for 12 hrs at 55°C, at 100 mbar to afford pure (R,S)-(V) [161g, c.y. 95%].

m.p. = 176°C.

¹H-NMR (300 MHz, CDCl₃, δ in ppm): 1.07 (d, 3H), 1.16 (d, 3H), 1.25 (d, 3H), 1.36 (d, 3H), 2.11 (s, 3H), 3.11 (d, 2H), 3.43 (br s, 1H), 4.04 (m, 1H), 4.99 (t, 1H), 6.63 (s, 1H), 6.83 (s, 2H), 7.29 (m, 5H), 9.10 (br s, 1H).

LC-MS (pos): 340.3 [M+H]⁺

### Example 30

### Preparation of (±)N,N-diisopropyl-3-(2-hydroxy-5-methylphenyl)-3-phenyl-2-propanamide (R,S)-(V)

Compound (II) (100 g, 0.297 mol), 10%Pd/C (0.5g, 50% wet) and methanol (1.0 L) are loaded into a 2 L autoclave, fitted with a mechanical stirrer, and put under hydrogen atmosphere (5 bar). The temperature is adjusted to 70°C and the reaction mixture stirred until complete conversion is reached (1h). The catalyst is filtered off from the hot mixture, and the filter cake washed with hot methanol (200 mL). The organic phase is diluted with water (100 mL) at 60-65°C under stirring to favor the crystallization of the product; then the mixture is cooled to 5°C, filtered and the solid washed with cold methanol /water (9/1) and dried under vacuum to afford pure (R,S)-(V) [88g, c.y. 88%].

### Example 31

### Preparation of (±) N,N-diisopropyl-3-(2-hydroxy-5-methylphenyl)-3-phenyl-2-propanamide (R,S)-(V)

Following the procedure of example 1 and treating the crude not isolated product (II) in 2-Me-THF, after a double treatment with charcoal, with 10% Pd/C and hydrogen at 5 bar at 70°C, the crude (R,S)-(V) is obtained.

### Example 32

### Preparation of (±) N,N-diisopropyl-3-(2-hydroxy-5-methylphenyl)-3-phenyl-2-propanamide (R,S)-(V)

An aqueous solution of hypophosphite is prepared by mixing water (1.0 L), sodium hypophosphite (85 g) and sodium carbonate (85 g) and stirred until dissolution. A 3 L round bottomed flask, fitted with a mechanical stirrer, is loaded with compound (II) (168 g, 0.499 mol), 2-Me-THF (2.0 L) and ¼ of the prepared hypophosphite solution. The flask is washed three times with nitrogen, 5%Pd/C (18g, 50% wet) is added and the reaction is warmed, under vigorously stirring, at 50-55°C. After 20' at this temperature, the rest of the hypophosphite solution is dropped over 1.5 hrs at 55°C. The mixture is filtered, at 55°C, trough a pad of celite and the filter is washed with wet, warm 2-Me-THF (180 mL). The aqueous phase is separated and the organic phase is washed two times with warm water (2 x 200 mL) maintaining the temperature at 50-55°C. The organic phase is treated as in previous example to afford 163 g of pure (R,S)-(V).

### Examples 33-35

### Preparation of N,N-diisopropyl-3-(2-hydroxy-5-methylphenyl)-3-phenyl-2-propanamide (R)-(V) and (S)-(V)

To a 50-mL stainless steel autoclave equipped with a Teflon coated stirrer bar is placed [RuCl(*p*-cymene)((*R*)-dm-segphos)]C1 (3.0 mg, 0.00296 mmol) and E-(II) (200 mg, 0.593 mmol). The atmosphere is replaced with nitrogen gas, followed by addition of THF-MeOH (10:1) (1.1mL) and THF-MeOH (10:1) solution (0.593 M) of TFA (0.1 mL, 0.0593 mmol). Hydrogen is initially introduced into the autoclave at a pressure of 1.0 MPa, before being reduced to 0.1 MPa by carefully releasing the stop valve. After this procedure is repeated three times, hydrogen is introduced at 3.0 MPa and the solution is stirred at 60°C for 16h. The reaction mixture is diluted with MeOH (6.8mL). 150µL of sample is diluted with IPA (ca 1.35mL) and analyzed by HPLC. 94.6 area % (91.6%ee) of (S)-(V) is observed.

Working with the same amount of [RuCl(*p*-cymene)((*R*)-dm-segphos)]Cl catalyst and (II), but using Z-isomer, in pure MeOH and in absence of TFA at 80°C for 16h, a HPLC conversion of 97 area % (87% e.e.) of (R)-(V) is observed.

Working with the same amount of [RuCl(*p*-cymene)((*R*)-dm-segphos)]Cl catalyst and (II), but using E-isomer, in pure MeOH and in absence of TFA at 80°C for 16h, a HPLC conversion of 98 area % (74% e.e.) of (S)-(V) is observed.

### Examples 36-37

### Preparation of N,N-diisopropyl-3-(2-hydroxy-5-methylphenyl)-3-phenyl-2-propanamide (R)-(V) and (S)-(V)

To a 100-mL stainless steel autoclave equipped with a Teflon coated stirrer bar is placed [Ru(S)-TMBTP(OCOCF₃)₂ (3.5 mg, 0.00432 mmol) and (II) as(E/Z 82/18) mixture (250 mg, 0.741 mmol). The atmosphere is replaced with nitrogen gas, followed by addition of EtOH (2.5 mL). Hydrogen is initially introduced into the autoclave at a pressure of 1.0 MPa, before being reduced to 0.1 MPa by carefully releasing the stop valve. After this procedure is repeated three times, hydrogen is introduced at 2.0 MPa and the solution is stirred at 50°C for 20h. The reaction mixture is analyzed by HPLC: 97 area % (35%ee) of (R)-(V) is observed.

Working with [Ru(R)-TMBTP(OCOCF3)2 (30mg) and (II) (1g, but using pure E isomer), in pure EtOH (10mL) at 50°C for 16 h, a HPLC conversion of 97 area % (41% e.e.) of (S)-(V) is observed.

### Example 38

### Preparation of N,N-diisopropyl-3-(2-Hydroxy-5-methylphenyl)-3-phenyl propylamine (R,S)-(I) as hydrochloride salt

A well dried 3 L round bottomed flask, fitted with a mechanical stirrer, is purged with nitrogen and loaded with compound (R,S)-(V) (50 g, 0.147 mol) and toluene (500 mL). The suspension is cooled with an ice bath and a first portion of Vitride^{™} (27g, ≥65% w/w in toluene) is added, dropwise, over 30 min., at 5-10°C (exothermic reaction and H₂ evolution occur). Then the second portion of Vitride^{™} (157 g, ≥65% w/w in toluene) is added, over 30 min, warming gently to 20-25°C. Then, the temperature is allowed to rise up to 30°C. This temperature is maintained for 3 hrs before warming the reaction to 40°C for 2h. Acetone (60 mL) is slowly dropped letting the temperature rise to 60-70°C, After 5 min 10%NaOH (150 mL) is added and the mixture is stirred for 15 min at 40-60°C, before cooling it to RT. Then, the aqueous phase is separated and the organic phase washed, in order, with dil. NaOH (5 mL 30% NaOH + 100 mL H2O), 7% aqueous NaHCO₃ (75 mL, until the pH of the discarded aqueous phase was 8-9) and finally with water (2 x 75 mL).

The final organic solution is evaporated under reduced pressure using a water bath (Tmax 50-60°C). The residue (about 54g) is dissolved in methyl-isobutyl-ketone (350 mL) and warmed to 50-60°C. 30% HCl is dropped (18 mL) and 100 mL of methyl-isobutyl-ketone are distilled, under reduced pressure. Then the mixture was cooled to 20°C in 1hr, stirred for 2 hrs at this temperature and filtered to afford (R,S)-(I) hydrochloride [49 g (c.y. 92%)].

### Example 39

### Preparation of N,N-diisopropyl-3-(2-hydroxy-5-methylphenyl)-3-phenyl propylamine (I) as L-tartrate salt

The reduction of 20 g compound (V) with Vitride ^{™} is carried out as in the previous example but the mixture, at the end of reaction, is worked as follows. The residue, obtained after work-up and evaporation of the solvent, is dissolved in 95% ethanol (50 mL) and warmed to 60-70°C. Tartaric acid (12 g) was dissolved in ethanol (125 mL) at 60-70 mL and the solution was added to the tolterodine solution, over 15 min, keeping the temperature in the 60-70°C range. The suspension was warmed to reflux, maintained for 1hr, cooled to RT in 1 hr and stirred overnight. Than, the mixture was cooled to 0-5°C and stirred for 10 hrs. The product is collected by filtration, washed with cold 95% ethanol (2 x 25 mL) and dried under vacuum to afford high pure compound (I) as L-tartrate [19.8 g (c.y.78%), assay: 99.4%.,(R)-(I)/(S)-(I) ratio=60/40. This product may be then crystallized in EtOH 95% according to a known procedure to afford highly pure (R)-(I) [c.y 37%, e.e.99.4%].

### Example 40

### Preparation of N,N-diisopropyl-3-(2-hydroxy-5-methylphenyl)-3-phenyl propylamine (I) as L-tartrate salt

The reduction of 50 g of compound (V) with Vitride^{™} is carried out and worked up as in the previous example to obtain crude (I) after the evaporation of solvent. The viscous residue (about 54g) is dissolved in isopropanol (500 mL) and the solution warmed 70-75°C. At this point, L-Tartaric acid (25 g) is added and the temperature is maintained for 30 min under stirring. The suspension is cooled to RT in 1hr and stirred for 2h. The product is filtered and washed with isopropanol to afford (I) as L-tartrate salt. Yield: 90%. (R)-(I)/(S)-(I) ratio=51/49.

### Example 41

### Preparation of N,N-diisopropyl-3-(2-hydroxy-5-methylphenyl)-3-phenyl propylamine (I) as L-tartrate salt

Working as in example 39, but using absolute ethanol, 22.9 g of compound (I) as L-tartrate were obtained [(c.y.82%), assay: 99.4%., (R)-(I)/(S)- (I) ratio=54/46].

### Example 42

### Comparative examples of partial enriched N,N-diisopropyl-3-(2-Hydroxy-5-methylphenyl)- 3-phenyl propylamine (I) as L-tartrate salt.

### A) starting from (R)-(I)/(S)- (I) (ratio=50/50) as HCl salt

40.4 g of racemic (I), obtained starting from the corresponding hydrochloride (45g, 0.124 mol), are diluted in 112 mL of EtOH 95%, heated at 60°C and added with a hot solution of 27.9g (1.5 eq) of L-Tartaric acid in 280 mL of EtOH 95%. The mixture is maintained at 60-70°c for 30', cooled in 1 h at RT, maintained overnight at RT, and then for 10h a 0-5°C, filtered and washed with 2x 90 mL of cold EtOH to afford 46.9 g of L-tartrate salt [(R)-(I)/(S)- (I) ratio=60/40].

### B) starting from (R)-(I)/(S)- (I) (ratio=51/49) as L-tartrate salt

59 g (0.124 mol) of this L-tartrate salt are diluted in 252 mL of EtOH 95%, heated at 60°C and added with a hot solution of 9.3 g (0.5 eq) of L-Tartaric acid in 140 mL of EtOH 95%. The mixture is maintained at 60-70°C for 30', cooled in 1 h at RT, maintained overnight at RT, and then for 10h a 0-5°C, filtered and washed with 2x 90 mL of cold EtOH to afford 35.6 g of L-tartrate salt [(R)-(I)/(S)- (I) ratio=82/18].

## Claims

1. N,N-Diisopropyl-3-(2-hydroxy-5-methylphenyl)-3-phenyl-2-propenamide of formula (II)

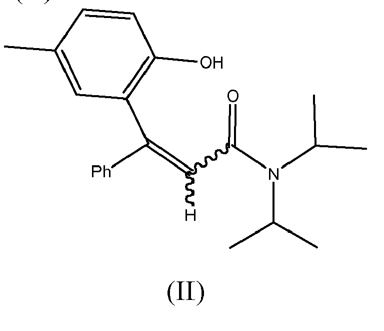

2. The compound of claim 1 as pure Z or E isomer or any possible combination of Z and E isomers.

3. A process for the preparation of the compound of claim 1 or 2 which comprises subjecting a compound of formula (III), where X is chloro, bromo or iodo, to a Mizoroki-Heck reaction with N,N-diisopropyl-3-phenyl-2-propenamide (IV)

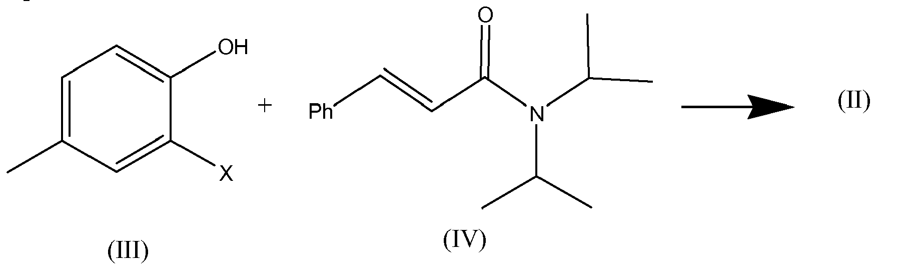

4. A process according to claim 3 wherein the reaction is carried out in the presence of a suitable catalyst and of a base.

5. A process according to claim 3 or 4 wherein the (IV)/(III) ratio is in the range from 2.5/1 to 0.9/1, preferably from 1.5/1 to 1/1.

6. A process according to any one of claims 3 to 5 wherein the reaction is performed in the presence of a medium or low polarity organic solvent.

7. A process according to any one of claims 4 to 6 wherein the base is a sterically hindered organic base, in a ratio to compound (III) from 1/1 to 1.5/1.

8. A process according to one or more of claims 4-7 wherein the catalyst is an homogeneous Pd(0) catalyst of formula Pd(0)L₂ + R₃P or Pd(0)(PR₃)₂, where L is a suitable ligand of palladium species and R is alkyl, cyclohexyl, aryl or heteroaryl group, prepared in situ or preformed in a separate vessel in a ratio to compound (III) from 100:1 1 to 10000:1.

9. A process according to claims 8 wherein L is dibenzylideneacetone.

10. A process according to claims 8 or 9 wherein Pd(0)L₂ is prepared by in situ by reduction of Pd(OAc).

11. A process according to claims 8-10 wherein R₃P is a hindered electron rich-phosphine selected from t.butylphosphine, di-t-butyl(2,2-diphenyl-1-methyl-1-cyclopropyl)phosphine (c-BRIDP) or di(1-adamantyl)-*n-*butylphosphine.

12. A process according to one or more of claims 3-11 wherein a higher content of the E isomer of compound (II) is obtained by carrying out the reaction either at a temperature lower than 100°C or at a temperature higher than 100°C but for a reaction time shorter than 20'.

13. A process according to one or more of claims 3-11 wherein a higher content of the Z isomer of compound (II) is obtained by carrying out the reaction at a temperature higher than 100°C for a reaction time longer than 20' .

14. A process according to one or more of claims 3-13 wherein the purity of E or Z isomer is increased by removing the unwanted isomer by selective crystallization and isolation of the solid E isomer in a solvent selected from toluene, 2-methyl-tetrahydrofuran, methanol, optionally in the presence of water, or by concentration of mother liquors if Z isomer is desired.

15. A process for the preparation of racemic or enantioenriched 3-(2-hydroxy-5-methylphenyl)-N,N-diisopropyl-3-phenyl propylamine (I) comprising the reduction of compound (II) by hydrogen or hydrogen donor or hydride in the presence of suitable catalyst followed by the reduction of the obtained racemic or enantioenriched N,N-diisopropyl-3-(2-hydroxy-5-methylphenyl)-3-phenyl propionamide (V)with an hydride.

16. A process according to claim 15 wherein the reduction of the compound (II) is performed with hydrogen or sodium hypophosphite in the presence of 3%, 5% or 10% wet Pd/C.

17. A process according to claim 15 wherein the reduction of the compound (II) is performed with hydrogen in the presence of a ruthenium complex containing non racemic chiral phosphine as ligands.

18. A process according to claim 17 which comprises:
(i) preparing N,N-diisopropyl-3-(2-hydroxy-5-methylphenyl)-3-phenyl-2-propenamide (II) by the Mizoroki-Heck reaction as described in claims 3-14;
(ii) reducing the intermediate (II) to obtain the saturated amide (V) as described in claims 16-17;
(iii) reducing the saturated amide (V) by a suitable hydride;
(iv) isolating 3-(2-hydroxy-5-methylphenyl)-N,N-diisopropyl-3-phenyl propylamine (I) as an acid addition salt.

19. A process according to the claim 18 wherein compound (I) is isolated as tartrate salt, particularly as (L)-tartrate salt.
